# EUROPEAN PATENT SPECIFICATION

(11) **EP 3 654 881 B1**
(45) Date of publication and mention of the grant of the patent: **07.08.2024**
(21) Application number: 17740399.5
(22) Date of filing: 18.07.2017
(51) Int. Cl.: A61F 2/24, G06T 7/00, A61B 34/20

(54) **ADJUSTABLE PERCUTANEOUS ANNULOPLASTY DEVICES AND DELIVERY SYSTEMS FOR PERCUTANEOUSLY DEPLOYING AN ANNULOPLASTY DEVICE AND A METHOD PERFORMED BY ONE OR MORE PROCESSING DEVICES**
ANPASSBARE PERKUTANE ANULOPLASTIEVORRICHTUNGEN UND FREISETZUNGSSYSTEME ZUM PERKUTANEN EINSATZ EINER ANULOPLASTIEVORRICHTUNG UND VON EINER ODER MEHREREN VERARBEITUNGSVORRICHTUNGEN DURCHGEFÜHRTES VERFAHREN
DISPOSITIFS D'ANNULOPLASTIE PERCUTANÉE AJUSTABLES ET SYSTÈMES DE MISE EN PLACE DE DÉPLOIEMENT PERCUTANÉ D'UN DISPOSITIF D'ANNULOPLASTIE ET PROCÉDÉ MIS EN OEUVRE PAR UN OU PLUSIEURS DISPOSITIFS DE TRAITEMENT

(43) Date of publication of application: 27.05.2020
(73) Proprietor: Kephalios S.A.S., 75008 Paris (FR)
(72) Inventor: TOZZI, Piergiorgio, 1005 Lausanne (CH); HAYOZ, Daniel, 1752 Villars-sur-Glâne (CH); GARD, Marco, 10031 Borgomasino (IT); ZANOTTI, Daniele, 13100 Aix en Provence (FR)
(74) Representative: Hepp Wenger Ryffel AG
(86) International application number: PCT/EP2017/068164
(87) International publication number: WO 2019/015747

(56) References cited:
- EP-A2- 1 749 475
- WO-A1-2010/150147
- WO-A2-2006/003576
- US-A1- 2006 155 184
- US-A1- 2007 055 368
- US-A1- 2007 233 239
- US-A1- 2008 177 382
- US-A1- 2012 296 417

## Description

The present invention relates to adjustable annuloplasty devices and delivery systems for deploying an annuloplasty device and a method performed by one or more processing devices. Some non-limiting examples focus on treating atrioventricular cardiac valves such as the mitral valve or the tricuspid valve, but the concept, function and benefit are not limited to these valves.

The performance of native valves, such as the mitral valve, may be reduced due to functional regurgitation. In functional regurgitation the ventricle (typically the left ventricle) is distorted or dilated. As a result the papillary muscles that support the leaflets of the native valve are stretched and the valve leaflets can no longer coapt properly.

Further, the performance of native valve may be reduced to a degenerative valve disease. Degenerative valve disease refers to a spectrum of conditions, in which morphologic changes in the connective tissue of the mitral valve cause structural lesions that prevent normal function of the mitral apparatus. One example of degenerative is annular dilation in the cross-section of the native valve.

Annuloplasty (e.g. mitral or tricuspid annuloplasty) is the implantation of an annuloplasty device (e.g., mitral ring or tricuspid ring) to deform and/or reinforce the valve annulus to correct insufficient valve function which may be caused by functional and/or degenerative regurgitation. During a classical annuloplasty procedure, the surgeon gauges the valve annulus and chooses a fixed size annuloplasty device accordingly. This procedure is performed in an open heart surgery on the arrested heart under cardiopulmonary bypass. However, open heart surgery and cardiopulmonary bypass impose significant risks on the patient. In particular, fragile patients have a high mortality rate in such surgeries. Furthermore open heart surgery requires prolonged hospital stays and additional care.

Document US2008177382 A1 discloses a medical device and methods for percutaneously treating a heart valve are described. The medical device has a first cardiac tissue fastener having a first lock, a second cardiac tissue fastener having a second lock, and a chord coupled to the first and second fasteners. Pulling the chord engages the first lock with the second lock.

A further limitation of the classical procedure or known trans-catheter annuloplasty devices is that, after implantation of an annuloplasty device, the size and geometry of the heart and the treated valve annulus may vary over time. For example, a dilated heart annulus may further widen or as in functional regurgitation the papillary muscles are further dilated. A fixed-size annuloplasty device may as a result become ineffective over time or inappropriate for the size of the valve annulus. This causes recurrent mitral regurgitation and poor clinical outcome. The common practice is to implant undersized rings in order to overcome the risk of recurrent regurgitation. Too small rings however, may result in hypertension which in turn leads to a further dilatation stress of the annulus. Furthermore, a small orifice area in the mitral valve may cause mitral stenosis.

WO 2016/174669 A1, filed by Valtech Cardio Ltd., discloses an annuloplasty structure having a primary body portion and a contraction member extending along a contracting portion of the annuloplasty structure. Further the structure comprises an actuatable adjustment mechanism coupled to the contraction member, that when actuated, adjusts a length of the annuloplasty structure by applying tension to the contraction member. The adjustment mechanism also includes a contraction-member-protecting element, having a first end coupled to the primary body portion of the annuloplasty structure, and a second end coupled to the adjustment mechanism. The contraction member extends from the adjustment mechanism via the contraction-member-protecting element to the primary body portion of the annuloplasty structure.

US 2016/0331534 A1, filed by Valcare, discloses a repair of heart valves through percutaneous trans-catheter delivery and fixation of annuloplasty rings to heart valves via a trans-apical approach to accessing the heart. A guiding sheath may be introduced into a ventricle of the heart through an access site at an apex of the heart. A distal end of the guiding sheath can be positioned retrograde through the target valve. An annuloplasty ring arranged in a compressed delivery geometry is advanced through the guiding sheath and into a distal portion of the guiding sheath positioned within the atrium of the heart. The distal end of the guiding sheath is retracted, thereby exposing the annuloplasty ring. The annuloplasty ring may be expanded from the delivery geometry to an operable geometry. Anchors on the annuloplasty ring may be deployed to press into and engage tissue of the annulus of the target valve.

WO 2015/121075 discloses an annuloplasty device which is adjustable. Three portions of an outer wall are more rigid than opposite portions of an inner wall. The inner wall is adapted to be displaced inwardly by an actuation element while the outer wall remains basically constant.

The problem of the invention is to provide a percutaneously implantable annuloplasty device, which allows an adjustment, i.e. fitting, of the annuloplasty device to the native valve annulus. In particular the problem of the invention is to increase coaptation between valve leaflets.

The invention is defined by the appended claims.

Non-limiting embodiments of the invention are described, by way of example only, with respect to the accompanying drawings, in which:
- Fig. 1:: is a schematic perspective view of a delivery system with an annuloplasty device during a first step of implantation of an annuloplasty device,
- Fig. 2:: is a first schematic perspective view of the delivery system with the annuloplasty device during a second step of implantation of an annuloplasty device,
- Fig. 3:: is a second schematic perspective view of the delivery system with the annuloplasty device during the second step of implantation of an annuloplasty device,
- Fig. 4:: is a first schematic perspective view of the delivery system with the annuloplasty device during a third step of implantation of an annuloplasty device,
- Fig. 5:: is a second schematic perspective view of the delivery system with the annuloplasty device during the third step of implantation of an annuloplasty device,
- Fig. 6:: is a first schematic perspective view of the delivery system with the annuloplasty device during a fourth step of implantation of an annuloplasty device,
- Fig. 7:: is a second schematic perspective view of the delivery system with the annuloplasty device during a fifth step of implantation of an annuloplasty device,
- Fig. 8:: is a first schematic perspective view of the delivery system with the annuloplasty device during a sixth step of implantation of an annuloplasty device,
- Fig. 9:: is a second schematic perspective view of the delivery system with the annuloplasty device during the sixth step of implantation of an annuloplasty device,
- Fig. 10:: is a schematic perspective view of the implanted annuloplasty device and
- Fig. 11:: is a schematic drawing of a mitral valve as seen from the left atrium.

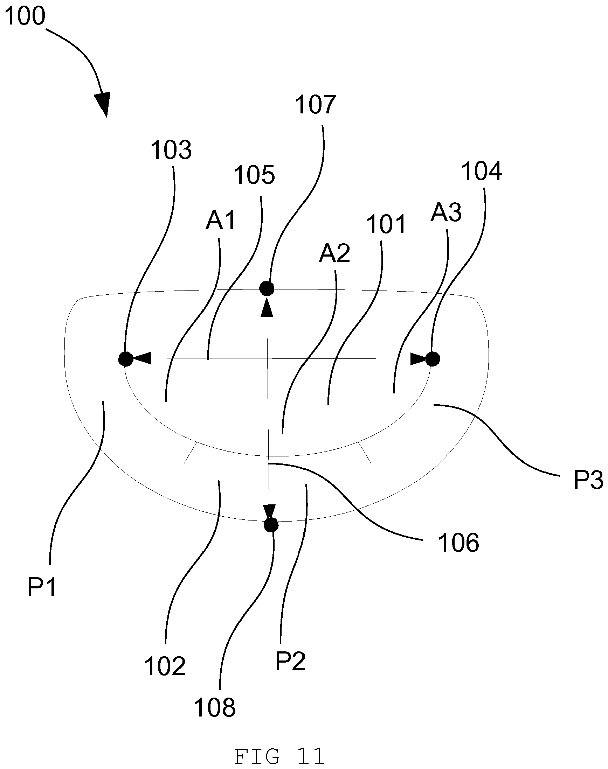

Figure 11 shows a schematic drawing of a mitral valve as seen from the left atrium. The mitral valve 100 comprises an anterior leaflet 101 and a posterior leaflet 102. Both leaflets 101, 102, extend into the left ventricle, where they co-apt. The leaflets 101, and 102, are biased towards the left ventricle and kept from prolapsing into the left atrium by chordae. Around the circumference of the mitral valve 100, the leaflets 101, 102 are integral with the valve annulus. The anterior and the posterior leaflet each comprise three scallops. The anterior scallops A1, A2, A3 and the posterior scallops P1, P2, P3 are positioned across each other such that in a healthy valve A1 and P1, A2 and P2, and, A3 and P3 coapt. Certain medical conditions may reduce a coaptation between the leaflets 101 and 102. As a result of the insufficient coaptation, there may be flow from the left ventricle to the left atrium. This type of blood flow is called regurgitation.

In order to inhibit the regurgitation, the valve annulus may be re-shaped. A position where the two leaflets end, 103 and 104, is called a commissure. A distance between the two commissures is an inter-commissural distance 105. Further, an anterior-posterior distance 106 is defined by the positions of two points 107 and 108. The point 107 corresponds to a position of the A2 scallop on the annulus. The point 108 corresponds to a position of the P2 scallop on the annulus.

To re-shape the annulus a physician can reduce the distance 106 between the anterior leaflet and the posterior leaflet to increase a coaptation area. Additionally the physician may also bring the commissures closer together, i.e. reduce the inter-commissural distance 105.

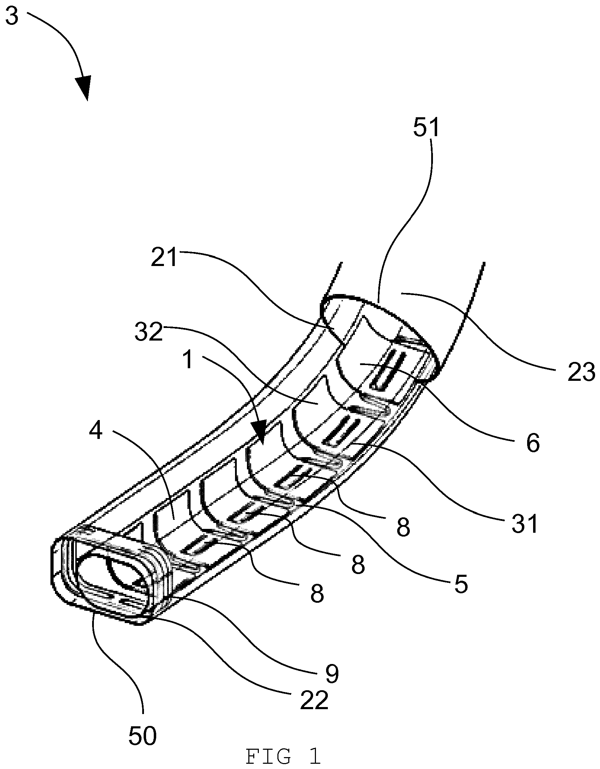

Figure 1 shows a schematic drawing of a delivery system 3 with an annuloplasty device 1 and a delivery device during a first step of implantation of the annuloplasty device 1. The delivery device includes a delivery tube 23, an outer sleeve 21 and an inner sleeve 22. Figure 1 shows the first step of implantation, in which the annuloplasty device 1 is released from a distal end 51 of the delivery tube 23. Within the delivery tube 23 the outer sleeve 21 and the inner sleeve 22 are disposed. The inner sleeve 22 is placed within the outer sleeve 21. The delivery tube 23 is relatively stiff compared to the inner and outer sleeves wherein inner and outer tubes are made of a softer material such that they may assume an annular shape. However, the delivery tube 23 is adapted for the transatrial approach. Thus the tube is flexible enough for the sharp turns in the aortic arch and through a left ventricle. In between the inner sleeve 22 and the outer sleeve 21, a first commissural anchor 9 is disposed at a distal end 50 of the outer tube 21. The first commissural anchor 9 is later placed in proximity to the anterior commissure 103 of the native valve and thus called anterior commissural anchor 9 in the following. Within the inner tube 22 a base 4 is held. The base 4 comprises of alternating rigid elements 6 and flexible elements 5.

Before inner and outer sleeve 21, 22 are released from the delivery tube 23, the delivery tube 23 is brought to a suitable location, i.e. a mitral valve annulus. The delivery system 3 is brought to the mitral valve annulus either through a trans-septal or a trans-femoral or a trans-apical approach. The trans-septal approach is preferred. Once a distal end 51 of the delivery tube 23 is in a suitable location, i.e. the mitral valve annulus, the release procedure of the annuloplasty device 1 is initiated by exteriorizing the inner and outer sleeves 21, 22 simultaneously as shown in figure 1.

The rigid element 6 includes a plate which comprises a lower pad 31 and an angled pad 32. In an implanted position, the lower pad 31 is anchored to tissue of a native valve annulus. The pads 31, 32 are made from the single plate which is bent in a middle part. The rigid elements 6 are interconnected by the flexible elements 5. As can be seen from figure 1, the lower pad 31 comprises a U-shaped cut out. The U-shaped cut out defines anchors 8.

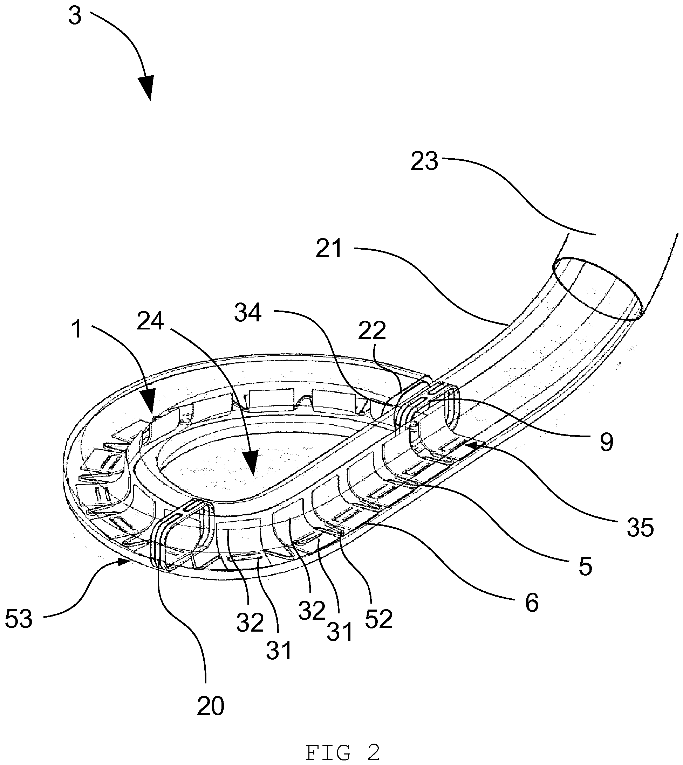

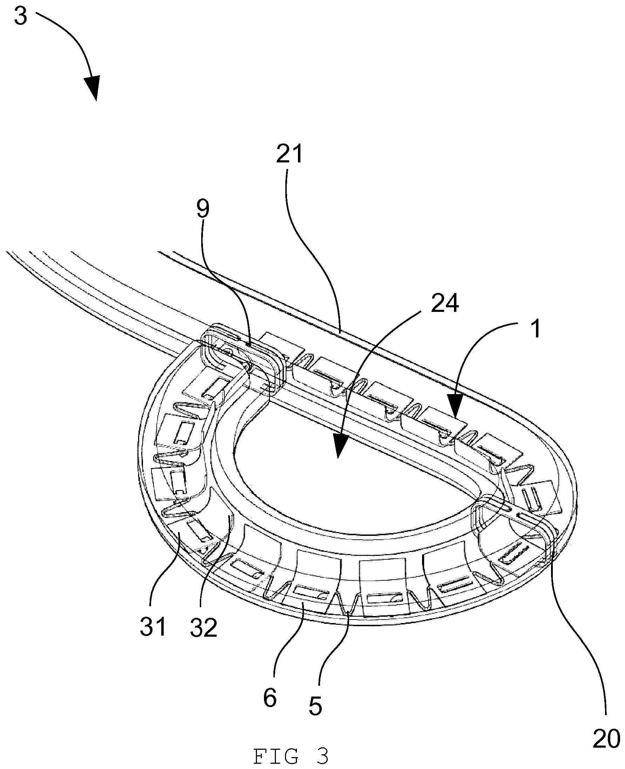

Figures 2 and 3 show a first and a second schematic view of the delivery system 3 with the annuloplasty device 1 during a second step of implantation. As inner and outer sleeves 21, 22 are further pushed out of the delivery tube 23, the entire base 4 is released from the delivery tube 23. The base 4 is made from a shape memory alloy nitinol. The shape memory alloy is biased such that upon release the base 4 assumes a D-shape.

Each flexible element 5 consists of a wire 52 which has an undulating shape. The wires 52 are made from a shape memory of material such that a selection of the wires expands. As a result, outer sleeve, inner sleeve and base assume a D-shape, once they are released from the stiffer delivery tube 23. The angled pad 32 faces towards the inner annular area 24. In the D-shape a distal end 34 of the base 4 and the anterior commissural anchor 9 are in contact which a proximal part 35 of the outer sleeve 21.

Further to the distally disposed commissural anchor 9, the delivery system comprises a second commissural anchor 20. The second commissural anchor 20 is located at an intermediary part 53 of the base is also disposed between inner and outer sleeve 21, 22. The second commissural anchor 20 is later placed in proximity to the posterior commissure 104 of the native valve and thus called posterior commissural anchor 20 in the following. Once the base 4 assumes the D-shape upon leaving delivery tube 23, the first and second commissural anchors are located at the "corners" of the D-shape.

During implantation the D-shape is aligned with the annulus of the mitral valve. The anterior commissural anchor 9 is arranged such that the anterior commissural anchor 9 is placed on the anterior commissure 103 of a native valve annulus. Then the outer sleeve 21 is retracted.

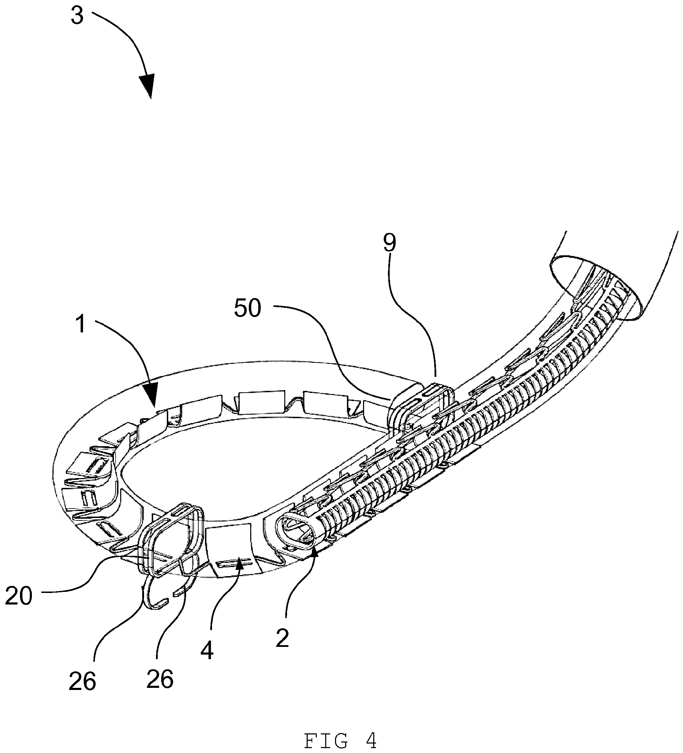

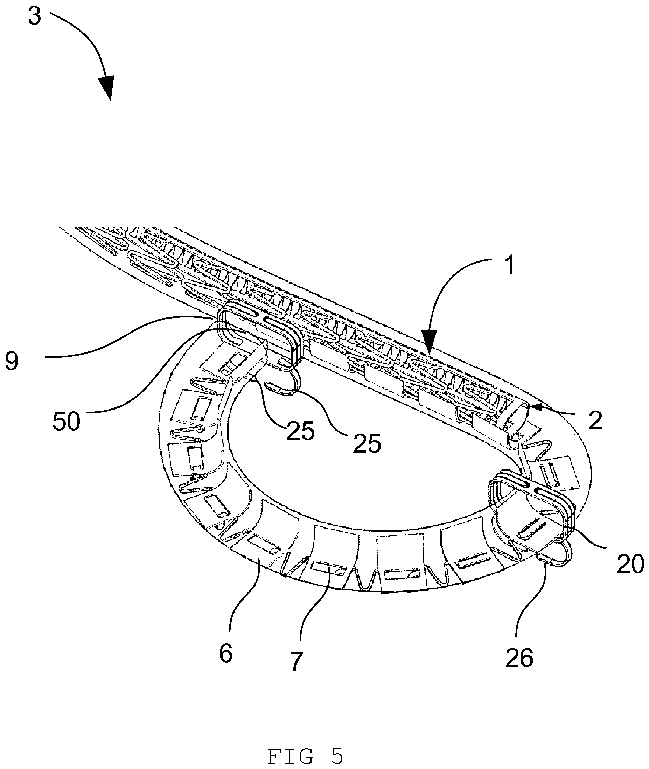

Figures 4 and 5 show a first and second schematic view of the delivery system 3 with the annuloplasty device 1 during a third step of implantation. Figures 4 and 5 show the delivery system 3 after the retraction of the outer sleeve 21. Both commissural anchors 9 and 20, are made of a shape memory alloy. The commissural anchors 9, 20 have a ring shape with cut outs. The cut outs extend a circumference of the commissural anchors 9 and 20 and define hooks 25, 26. As long as the commissural anchors 9 and 20 are held in the outer sleeve 21, these fingers are held within the annular shape of the commissural anchors 9 and 20. However, after retraction of the outer sleeve, the hooks 25, 26 bend out of the annular shape and fixate the commissural anchors 9, 20 in a tissue of the natural valve annulus. The retraction of the outer sleeve 21 is made in two steps. In a first step, under imaging guidance and after the anterior commissural anchor 9 is aligned with the anterior commissure 103 of the mitral valve (see fig. 11), the anterior commissural anchor 9 is released from the distal end 50 of the outer sleeve 21. Then, a position of the posterior commissural anchor 20 is found by pulling or pushing the delivery system forwards or backwards until the posterior commissural anchor 20 is in a desired position. Thus, while the anterior commissural anchor 9 is in a fixed position, the position of the posterior commissural anchor 20 may still be freely defined. Thereby, an inter-commissural distance 105 may be set according to a desired geometry after the annuloplasty. Once a suitable position is found, the outer sleeve 21 is fully retracted. Thereby, the hooks 26 of the posterior commissural anchor 20 are also released.

Then in a next step, the anterior-posterior distance is adjusted for a first time. Base 4 is fixedly attached to the first commissural anchor 9. Base 4 is not fixedly attached to the posterior commissural anchor 20. The base extends through the ring shape posterior commissural anchor 20. Thus, the base may be pushed distally and proximally through the posterior commissural anchor 20 which acts as a gate. The interior posterior distance is then adjusted by pulling or pushing the inner sleeve creating a smaller or larger radius of a round part of the D-shape. Thereby, the size of the native valve annulus is adjusted. Thereafter a cage 2 is advanced through the inner sleeve 22.

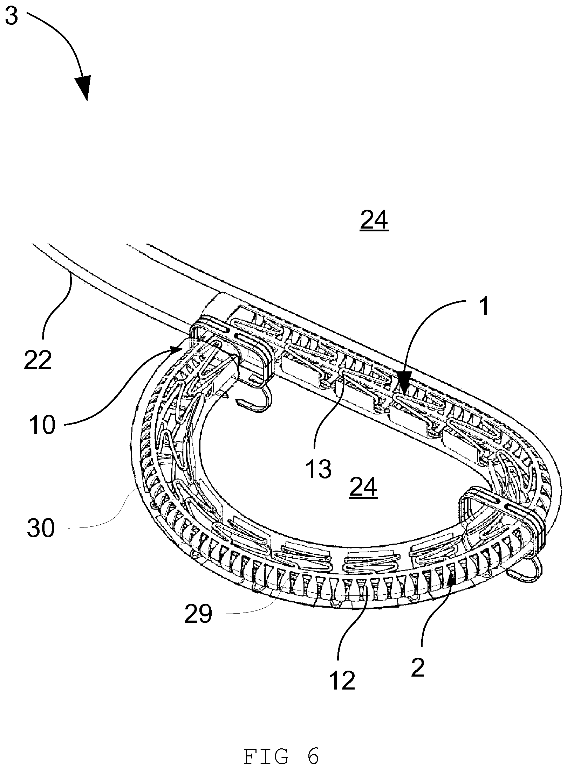

Figure 6 shows a schematic view of the delivery system 3 and the annuloplasty device 1 during a fourth step of implantation of the annuloplasty device 1. The cage 2 is advanced until its distal end 10 is distally beyond a distal end of the base. Figure 6 shows the delivery system 3, once the cage 2 has been fully advanced in the inner sleeve 22. As can be seen from figures 4 and 5, the base 4 has an L-shape, which covers a part of an inner surface of the inner sleeve 22.

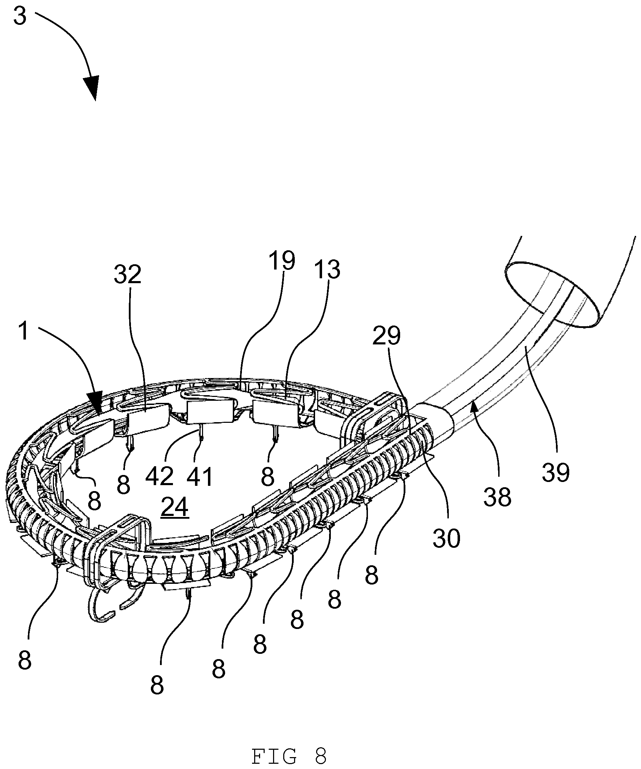

The cage 2 has a tubular shape which defines an inner lumen. Thus, the cage 2 may be advanced through the lumen of the inner sleeve 22 while the base is in the lumen. The cage 2 comprises an outer wall 12 and an inner wall 13. Inner and outer wall 12, 13 are defined with regard to the annular shape of the annuloplasty device 1. Thus the inner wall 13 is a part of the cage 2 which faces an inner annular area 24 and the outside wall as a part of the wall which faces an outside area 15. The outer wall 12 is defined by two longitudinally extending struts 29, which are interconnected by scales 30. The longitudinally extending struts 29 define a length of the cage 2 and prohibit an extension and/or compression the base 4. The inner wall 13 comprises undulating struts which extend around the circumference of the tubular cage. These undulating struts may be expanded towards the inner annular area, as will be explained in greater details in figures 8 to 10.

Like the base 4, the cage 2 is made from a nitinol and assumes upon exteriorization of the delivery tube 23 a D-shape, which facilitates advancing the cage 2.

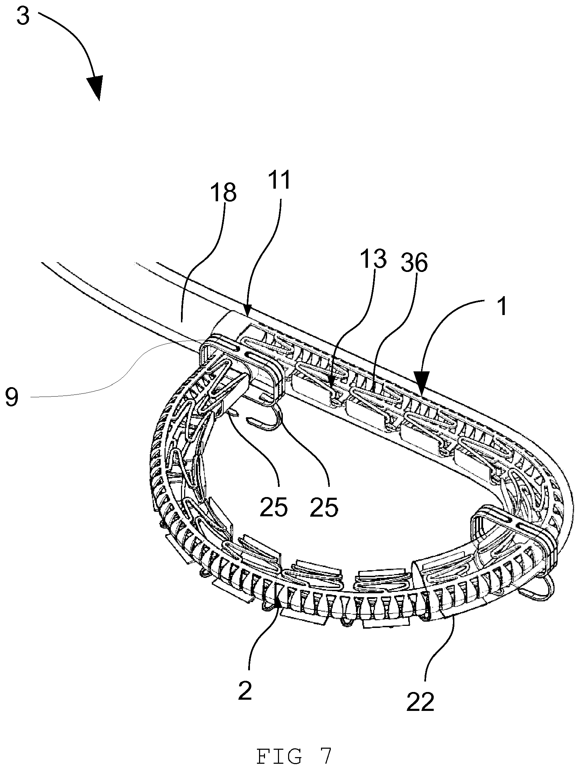

Figure 7 shows a schematic view of the delivery system of the annuloplasty device during a fifth step of implantation of the annuloplasty device 1. In figure 7 the inner sleeve 22 is partially retracted. The cage is advanced with a transmission line 18, which is attached to a proximal end 11 of the cage 2.

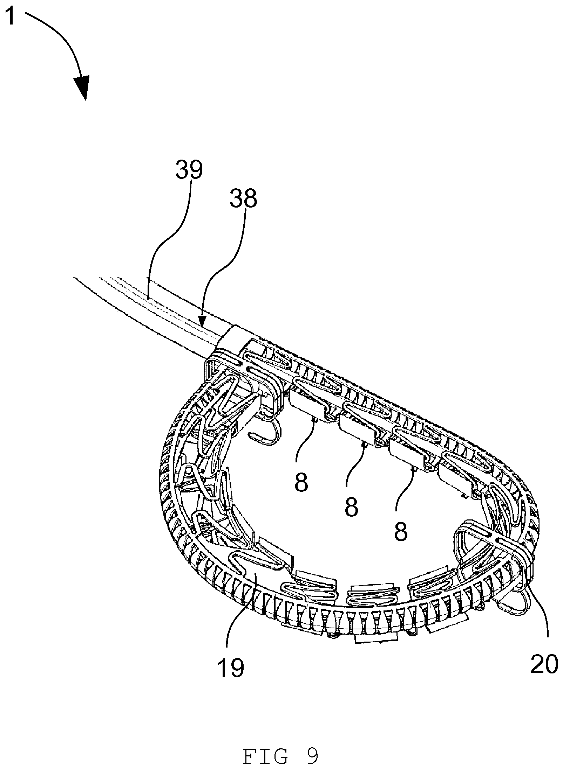

Figures 8 and 9 show a first and a second schematic view of the delivery system 3 with the annuloplasty device 1 during a sixth step of implantation of the annuloplasty device 1. Once cage 2 is fully advanced, the inner sleeve 22 may be withdrawn. In figure 7 the inner sleeve 22 is partially withdrawn. During withdrawal of inner sleeve 22 anchors 8 are deployed. The anchors include an elongate body part 42 and a sharp tip 41. When the anchors are deployed, they pivot outwardly into the tissue of the native valve.

During deployment the native valve annulus may be shaped. The anchors are deployed starting from a distal end. After first anchor 8 is placed a user pushes and/or pulls the delivery system to adjust a shape of the annulus. Then a second anchor is deployed. After the second anchor 8 is deployed the user may push or pull the delivery system again to align a position of the second anchor the corresponding tissue. The following anchors 8 are deployed in the same fashion until all anchors 8 are anchored in the native valve annulus and the native valve annulus is reshaped. During deployment, the anchors 8 are deployed along an inner edge of the annuloplasty device.

When the inner sleeve is retracted beyond the distal end of the base 4 and the cage 2, the distal end of the base 4 or the cage 2 or the anterior commissural anchor 9 interlocks with a proximal portion of the base or cage.

Once all anchors 8 are deployed, an initial shape of the annulus is set. This reshaping may be sufficient in order to inhibit regurgitation. However, if there is still residual regurgitation the annuloplasty device includes further adjustability. Furthermore, some patients, functional patients develop regurgitation after months or years of implantation of the annuloplasty device.

In both cases, the device is adjusted by advancing a balloon catheter 38 through the tubular transmission line 18 into the tubular shape of the cage 2. The balloon catheter 38 comprises a balloon 19 and a tube 39 through which the balloon 19 may be inflated. As can be seen from figures 8 and 9, the outer wall 12 with its longitudinal struts 29 and scales 30 has a higher rigidity than the inner wall 13 with its undulating struts. Thus, if the balloon 19 of the balloon catheter 38 is expanded, the inner wall 13 is pushed towards the inner annular area 24.

The anchors 8 are deployed along an inner edge of the annuloplasty device 1. Upon expanding the balloon catheter 38, the anchors 8 and the annular tissue in which they are anchored are moved simultaneously inwardly.

Thereby, the angled pad 32 and with it the lower pad 31 and also anchors 8 are also pushed towards the inner annular area 24. As a result, the annuloplasty is adjusted by pushing only a section towards the inner annular area. The inner wall 13 is less rigid than the outer wall 12 along its entire length and thus the annuloplasty device 1 is adjusted around its entire annular shape is desired until there is no residual regurgitation.

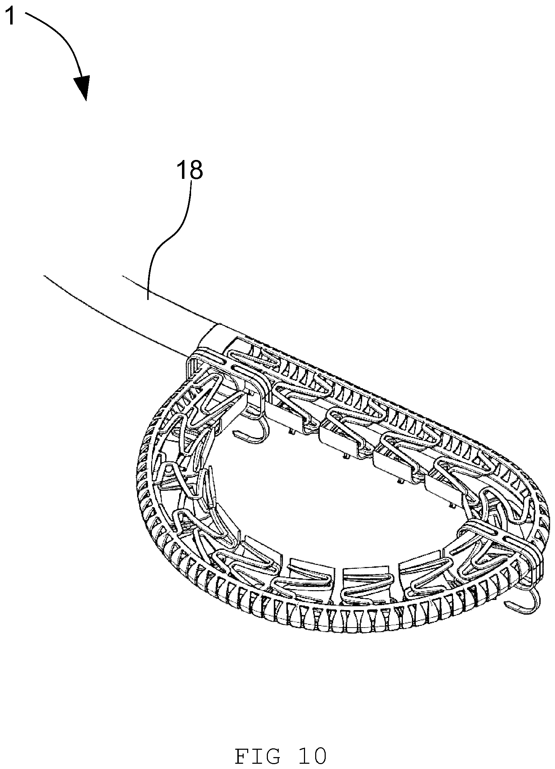

Figure 10 shows a schematic view of the implanted annuloplasty device. In figure 10 the balloon catheter 39 is removed from the annuloplasty device 1. Figure 10 shows a final implanted shape of the implanted annuloplasty device 1. A transmission line 18 is attached to the cage. The transmission line 18 is a tube whose inner lumen is distally connected to a cage.

A user may insert a balloon catheter again into the transmission at a later time to readjust the annuloplasty device 1, if necessary.

A distal part of the delivery tube 23 comprises a linear encoder. The linear encoder measures a relative position between delivery tube 23 and outer sleeve 21. Also a second linear encoder measures a relative position between the inner sleeve 22 and the delivery tube 23. A third encoder measures a relative position between the transmission line 18 and the balloon catheter 39. During implantation the measured relative positions are displayed to a surgeon. The relative positions indicate to the surgeon where the respective device is located, how much further they need to be advanced and in which shape the annuloplasty device currently is.

To aid the surgeon, their positions are visualised in a 3-D animation based on the shape memory properties of the annuloplasty device. The 3-D animation shows how the shape memory parts of the annuloplasty device assume their form without the need of further imaging technology. The 3-D animation shows the measured position in combination with 3-D model data with an imaging fusion.

Furthermore the 3-D data may be combined with imaging data from an echocardiography system. The echocardiography system provides fluoroscopic imaging data. The fluoroscopic imaging data is overlapped with the 3-D animation providing an augmented reality image or stream.

## Claims

1. An adjustable percutaneous annuloplasty device (1) comprising:
- a tubular, longitudinally extending cage (2) having a basically annular shape or being adapted to be brought into an annular shape upon release from a delivery device (3),
- a separate, longitudinally extending base (4) having at least a part of the basically annular shape of the cage (2) or being adapted to be brought into at least partially into the annular shape of the cage (2) upon release from a delivery device,
- wherein the cage (2) comprises anchors (8) for connecting the cage (2) to the annular base (4) and
- wherein the base (4) comprises at least one flexible element such that the annular shape of the base (4) is adjustable, and
- wherein the flexible element (5) allows an expansion and/or compression of the base along a longitudinal direction of the base (4).

2. Adjustable annuloplasty device (1) according to claim 1, **characterized in that** the cage (2) and the base are independently insertable to an implantation site and adapted to be connected to each other at the implantation site.

3. Adjustable annuloplasty device (1) according to claim 1 or 2, **characterized in that** the base additionally comprises at least two rigid elements (6) which are connected by a flexible element (5).

4. Adjustable annuloplasty device (1) according to claim 3, **characterized in that** the base (4) comprises alternating rigid and flexible elements (5, 6).

5. Adjustable annuloplasty device (1) according to one of the claims 3 to 4, **characterized in that** the at least one rigid element (6) is arranged movably within the base (4) such that it is pushable towards an inner annular area (24).

6. Adjustable annuloplasty device (1) according to one of the claims 3 to 5, **characterized in that** the at least one rigid element (6) includes an interface, preferably an opening, for a connection to the cage (2).

7. Adjustable annuloplasty device (1) according to one of the preceding claims, **characterized in that** the cage (2) is adjustable, wherein the cage comprises an outer and an inner wall (12, 13) and at least one portion of the outer wall (12) is more rigid than opposite portion(s) of the inner wall (13), wherein the inner wall (13) is arranged nearer to an inside area (24) defined by the annular shape than the outer wall (12), such that the inner wall (13) is adapted to be displaced inwardly at least along less rigid portion(s) of the circumference upon actuation by at least one actuation element while the outer wall (12) remains basically constant.

8. Adjustable annuloplasty device (1) according to claim 7, **characterized in that** the outer wall (12) is more rigid along its entire length than the at least a part of the, preferably the entire, length of the inner wall (13).

9. Adjustable annuloplasty device (1) according to one of the preceding claims, **characterized in that** the cage (2) comprises anchors (8) for engaging an annulus.

10. Adjustable annuloplasty device (1) according to one of the preceding claims, **characterized in that** the base (4) comprises anchors (8) for engaging an annulus and/or for connecting the cage (2) to the annular base (4).

11. Adjustable annuloplasty device (1) according to claim 9 or 10, **characterized in that** the anchors (9, 20) are made from or comprise a shape memory alloy.

12. Adjustable annuloplasty device (1) according to claim 11, **characterized in that** the anchors (8) comprise pins (40) with a longitudinally extending, preferably bent, body part (42) and a preferably sharp tip (41).

13. Adjustable annuloplasty device (1) according to one of the claims 3 to 6 and one of the claims 9 to 12, **characterized in that** the at least one rigid element (6) includes an opening, wherein the anchors (8) extend through or are adapted to extend through said opening.

14. Adjustable annuloplasty device (1) according to one of the preceding claims, **characterized in that** a distal end of the cage (2) and/or base (4) is spaced apart from a proximal section of the cage (2) and/or base (4).

15. Delivery system (3) comprising an annuloplasty device (1) according to one of the preceding claims, **characterized in that** the delivery system (1) additionally comprises a transmission line (18), preferably a catheter, for adjusting the cage (2) and/or annulus base (4), wherein the transmission line (18) preferably comprises a lumen for a balloon catheter (38).

16. An adjustable percutaneously annuloplasty device (1) according to one of the claims 1 to 14, comprising:
a longitudinally extending base (4) having a basically annular shape and a deployed state and an undeployed state,
wherein the base (4) is flexible such that the annular shape of the base (4) is adjustable during and/or after implantation, **characterized in that** the base (4) is made from or comprises a shape memory alloy such that in the deployed state upon release from a delivery device the base has an annular shape, preferably a D-shape.

17. Adjustable annuloplasty device (1) according to claim 16, **characterized in that**, the base includes at least one flexible element (5) and at least two rigid elements (6), which are connected by the flexible element (5) wherein the flexible element (5) allows an expansion and/or compression along a longitudinal direction of the base (4).

18. Adjustable annuloplasty device (1) according to claim 17 , **characterized in that** the base (4) comprises alternating rigid and flexible elements (5, 6).

19. Adjustable annuloplasty device (1) according to one of the claims 17 to 18, **characterized in that** the at least one rigid element (6) is moveable arranged within the base (4) such that it is pushable towards an inner annular area (24) .

20. Adjustable annuloplasty device (1) according to one of the claims 17 to 19, **characterized in that** the at least one rigid element (6) includes an interface, preferably an opening, for a connection to a cage (2).

21. Adjustable annuloplasty device (1) according to one of the claims 17 to 20, **characterized in that** the rigid element comprises a first pad (31), which extends along a plane of the annular shape and a second pad (32) which is angled relative to the first pad (31), wherein the second pad (32) preferably defines an inner annular area (24) of the annular shape.

22. Adjustable annuloplasty device (1) according to claim 17, **characterized in that** the flexible element comprises or consists of a wire, wherein the wire has an undulating shape, and wherein the wire (52) comprises a first and second end and each end is connected to a first surface of a rigid element (6).

23. Adjustable annuloplasty device (1) according to one of the preceding claims, **characterized in that** the annuloplasty device (1) comprises at least one, preferably two, commissural anchor (9, 20) for fixating the base (4) to an annulus.

24. Adjustable annuloplasty device (1) according to claim 23, **characterized in that** the commissural anchors (9, 20) is made from or comprises a shape memory alloy such that once a sleeve (21) covering the commissural anchor (9, 20) is removed, the anchors (9, 20) are deployed and adapted to penetrate the native tissue of the annulus so to realize two commissural fixations.

## Patentansprüche

1. Eine einstellbare perkutane Anuloplastievorrichtung (1), umfassend::
- ein röhrenförmiger, sich in Längsrichtung erstreckender Käfig (2), der eine im wesentlichen ringförmige Form aufweist oder beim Lösen aus einer Zuführungseinrichtung (3) in eine ringförmige Form gebracht werden kann,
- einen separaten, sich in Längsrichtung erstreckenden Basisteil (4), der zumindest einen Teil der im Wesentlichen ringförmigen Form des Käfigs (2) aufweist oder bei der Entnahme aus einer Zuführungseinrichtung zumindest teilweise in die ringförmige Form des Käfigs (2) gebracht werden kann,
- wobei der Käfig (2) Anker (8) zum Verbinden des Käfigs (2) mit dem ringförmigen Basisteil (4) aufweist und
- wobei das Basisteil (4) mindestens ein flexibles Element aufweist, so dass die Ringform des Basisteils (4) einstellbar ist, und
- wobei das flexible Element (5) eine Ausdehnung und/oder Kompression des Basisteils entlang einer Längsrichtung des Basisteils (4) ermöglicht.

2. Einstellbare Annuloplastievorrichtung (1) nach Anspruch 1, **dadurch gekennzeichnet, dass** der Käfig (2) und das Basisteil unabhängig voneinander in eine Implantationsstelle einführbar sind und an der Implantationsstelle miteinander verbunden werden können.

3. Einstellbare Annuloplastievorrichtung (1) nach Anspruch 1 oder 2, **dadurch gekennzeichnet, dass** das Basisteil zusätzlich aus mindestens zwei starren Elementen (6) besteht, die durch ein flexibles Element (5) verbunden sind.

4. Einstellbare Annuloplastievorrichtung (1) nach Anspruch 3, **dadurch gekennzeichnet, dass** das Basisteil (4) abwechselnd starre und flexible Elemente (5, 6) umfasst.

5. Einstellbare Annuloplastievorrichtung (1) nach einem der Ansprüche 3 bis 4, **dadurch gekennzeichnet, dass** das mindestens eine starre Element (6) innerhalb des Basisteils (4) beweglich angeordnet ist, so dass es gegen einen inneren Ringbereich (24) schiebbar ist.

6. Einstellbare Annuloplastievorrichtung (1) nach einem der Ansprüche 3 bis 5, **dadurch gekennzeichnet, dass** das mindestens eine starre Element (6) eine Schnittstelle, vorzugsweise eine Öffnung, für eine Verbindung mit dem Käfig (2) aufweist.

7. Einstellbare Annuloplastievorrichtung (1) nach einem der vorhergehenden Ansprüche, **dadurch gekennzeichnet, dass** der Käfig (2) einstellbar ist, wobei der Käfig eine äußere und eine innere Wand (12, 13) aufweist und mindestens ein Abschnitt der äußeren Wand (12) steifer ist als der oder die gegenüberliegenden Abschnitte der inneren Wand (13), wobei die innere Wand (13) näher an einem durch die Ringform definierten Innenbereich (24) angeordnet ist als die äußere Wand (12), so dass die innere Wand (13) so angepasst ist, dass sie bei Betätigung durch mindestens ein Betätigungselement zumindest entlang weniger starrer(r) Abschnitte des Umfangs nach innen verschoben wird, während die äußere Wand (12) im Wesentlichen konstant bleibt.

8. Einstellbare Annuloplastievorrichtung (1) nach Anspruch 7, **dadurch gekennzeichnet, dass** die Außenwand (12) über ihre gesamte Länge steifer ist als zumindest ein Teil der, vorzugsweise die gesamte, Länge der Innenwand (13).

9. Einstellbare Annuloplastievorrichtung (1) nach einem der vorhergehenden Ansprüche, **dadurch gekennzeichnet, dass** der Käfig (2) Anker (8) zum Eingriff in einen Annulus umfasst.

10. Einstellbare Annuloplastievorrichtung (1) nach einem der vorhergehenden Ansprüche, **dadurch gekennzeichnet, dass** das Basisteil (4) Anker (8) zum Eingriff in einen Annulus und/oder zur Verbindung des Käfigs (2) mit dem ringförmigen Basisteil (4) aufweist.

11. Einstellbare Annuloplastievorrichtung (1) nach Anspruch 9 oder 10, **dadurch gekennzeichnet, dass** die Anker (9, 20) aus einer Formgedächtnislegierung hergestellt sind oder diese umfassen.

12. Einstellbare Annuloplastievorrichtung (1) nach Anspruch 11, **dadurch gekennzeichnet, dass** die Anker (8) Stifte (40) mit einem sich in Längsrichtung erstreckenden, vorzugsweise gebogenen Körperteil (42) und einer vorzugsweise scharfen Spitze (41) umfassen.

13. Einstellbare Annuloplastievorrichtung (1) nach einem der Ansprüche 3 bis 6 und einem der Ansprüche 9 bis 12, **dadurch gekennzeichnet, dass** das mindestens eine starre Element (6) eine Öffnung aufweist, durch die sich die Anker (8) erstrecken oder erstrecken können.

14. Einstellbare Annuloplastievorrichtung (1) nach einem der vorhergehenden Ansprüche, **dadurch gekennzeichnet, dass** ein distales Ende des Käfigs (2) und/oder Basisteils (4) von einem proximalen Abschnitt des Käfigs (2) und/oder Basisteils (4) beabstandet ist.

15. Zuführungseinrichtung (3) mit einer Annuloplastieeinrichtung (1) nach einem der vorhergehenden Ansprüche, **dadurch gekennzeichnet, dass** die Zuführungseinrichtung (1) zusätzlich eine Transmissionsleitung (18), vorzugsweise einen Katheter, zur Verstellung des Käfigs (2) und/oder des Annulus-Basisteils (4) umfasst, wobei die Transmissionsleitung (18) vorzugsweise ein Lumen für einen Ballonkatheter (38) aufweist.

16. Einstellbare Annuloplastievorrichtung (1) nach einem der Ansprüche 1 bis 14, umfassend:
ein sich in Längsrichtung erstreckendes Basisteil (4) mit einer im Wesentlichen ringförmigen Form und einem ausgefahrenen Zustand und einem nicht ausgefahrenen Zustand, wobei das Basisteil (4) flexibel ist, so dass die ringförmige Form des Basisteils (4) während und/oder nach der Implantation einstellbar ist, **dadurch gekennzeichnet, dass** das Basisteil (4) aus einer Formgedächtnislegierung hergestellt ist oder diese umfasst, so dass das Basisteil im ausgefahrenen Zustand nach dem Lösen von einer Zuführungseinrichtung eine ringförmige Form, vorzugsweise eine D-Form, aufweist.

17. Einstellbare Annuloplastievorrichtung (1) nach Anspruch 16, **dadurch gekennzeichnet, dass** das Basisteil mindestens ein flexibles Element (5) und mindestens zwei starre Elemente (6) umfasst, die durch das flexible Element (5) verbunden sind, wobei das flexible Element (5) eine Ausdehnung und/oder Kompression entlang einer Längsrichtung des Basisteils (4) ermöglicht.

18. Einstellbare Annuloplastievorrichtung (1) nach Anspruch 17, **dadurch gekennzeichnet, dass** das Basisteil (4) abwechselnd starre und flexible Elemente (5, 6) aufweist.

19. Einstellbare Annuloplastievorrichtung (1) nach einem der Ansprüche 17 bis 18, **dadurch gekennzeichnet, dass** das mindestens eine starre Element (6) innerhalb des Basisteils (4) beweglich angeordnet ist, so dass es in Richtung eines inneren ringförmigen Bereichs (24) schiebbar ist.

20. Einstellbare Annuloplastievorrichtung (1) nach einem der Ansprüche 17 bis 19, **dadurch gekennzeichnet, dass** das mindestens eine starre Element (6) eine Schnittstelle, vorzugsweise eine Öffnung, für eine Verbindung mit einem Käfig (2) aufweist.

21. Einstellbare Annuloplastievorrichtung (1) nach einem der Ansprüche 17 bis 20, **dadurch gekennzeichnet, dass** das starre Element ein erstes Pad (31), das sich entlang einer Ebene der Ringform erstreckt, und ein zweites Pad (32) umfasst, das relativ zum ersten Pad (31) abgewinkelt ist, wobei das zweite Pad (32) vorzugsweise einen inneren Ringbereich (24) der Ringform definiert.

22. Einstellbare Annuloplastievorrichtung (1) nach Anspruch 17, **dadurch gekennzeichnet, dass** das flexible Element einen Draht umfasst oder aus einem Draht besteht, wobei der Draht eine wellenförmige Form aufweist und wobei der Draht (52) ein erstes und ein zweites Ende umfasst und jedes Ende mit einer ersten Oberfläche eines starren Elements (6) verbunden ist.

23. Einstellbare Annuloplastievorrichtung (1) nach einem der vorhergehenden Ansprüche, **dadurch gekennzeichnet, dass** die Annuloplastievorrichtung (1) mindestens einen, vorzugsweise zwei, Kommissuranker (9, 20) zur Fixierung des Basisteils (4) an einem Annulus aufweist.

24. Einstellbare Annuloplastievorrichtung (1) nach Anspruch 23, **dadurch gekennzeichnet, dass** die Kommissuranker (9, 20) aus einer Legierung mit Formgedächtnis hergestellt sind oder eine solche Legierung umfassen, so dass die Anker (9, 20), sobald eine Hülse (21), die den Kommissuranker (9, 20) bedeckt, entfernt ist, entfaltet werden und geeignet sind, das native Gewebe des Annulus zu durchdringen, um zwei Kommissurbefestigungen zu realisieren.

## Revendications

1. Dispositif d'annuloplastie percutanée ajustable (1) comprenant :
- une cage (2) tubulaire, s'étendant longitudinalement, ayant une forme essentiellement annulaire ou conçue pour prendre une forme annulaire lorsqu'elle est libérée d'un dispositif de délivrance (3),
- une base (4) séparée, s'étendant longitudinalement, ayant au moins une partie de la forme essentiellement annulaire de la cage (2) ou étant adaptée pour être amenée au moins partiellement dans la forme annulaire de la cage (2) lors de la libération d'un dispositif de délivrance,
- dans lequel la cage (2) comprend des ancrages (8) pour relier la cage (2) à la base annulaire (4) etdans lequel la base (4) comprend au moins un élément flexible de sorte que la forme annulaire de la base (4) est ajustable, et
- dans lequel l'élément flexible (5) permet une expansion et/ou une compression de la base le long d'une direction longitudinale de la base (4).

2. Dispositif d'annuloplastie ajustable (1) selon la revendication 1, **caractérisé en ce que** la cage (2) et la base sont insérables indépendamment jusqu'à un site d'implantation et adaptées pour être reliées l'une à l'autre au niveau du site d'implantation.

3. Dispositif d'annuloplastie ajustable (1) selon la revendication 1 ou 2, **caractérisé en ce que** la base comprend en outre au moins deux éléments rigides (6) reliés par un élément flexible (5).

4. Dispositif d'annuloplastie ajustable (1) selon la revendication 3, **caractérisé en ce que** la base (4) comprend des éléments (5, 6) en alternance rigides et flexibles.

5. Dispositif d'annuloplastie ajustable (1) selon l'une des revendications 3 à 4, **caractérisé en ce que** le au moins un élément rigide (6) est disposé de manière mobile à l'intérieur de la base (4) de façon à pouvoir être poussé vers une zone annulaire interne (24).

6. Dispositif d'annuloplastie ajustable (1) selon l'une des revendications 3 à 5, **caractérisé en ce que** le au moins un élément rigide (6) comprend une interface, de préférence une ouverture, pour une connexion à la cage (2).

7. Dispositif d'annuloplastie ajustable (1) selon l'une des revendications précédentes, **caractérisé en ce que** la cage (2) est ajustable, la cage comprenant une paroi extérieure et une paroi intérieure (12, 13) et au moins une portion de la paroi extérieure (12) étant plus rigide que la ou les portions opposées de la paroi intérieure (13), dans lequel la paroi intérieure (13) est disposée plus près d'une zone intérieure (24) définie par la forme annulaire que la paroi extérieure (12), de sorte que la paroi intérieure (13) est adaptée pour être déplacée vers l'intérieur au moins le long de portion(s) moins rigide(s) de la circonférence lors de l'actionnement par au moins un élément d'actionnement, tandis que la paroi extérieure (12) reste essentiellement constante.

8. Dispositif d'annuloplastie ajustable (1) selon la revendication 7, **caractérisé en ce que** la paroi externe (12) est plus rigide sur toute sa longueur que la paroi interne (13) sur au moins une partie de sa longueur, de préférence sur toute sa longueur.

9. Dispositif d'annuloplastie ajustable (1) selon l'une des revendications précédentes, **caractérisé en ce que** la cage (2) comprend des ancres (8) pour s'engager dans un anneau.

10. Dispositif d'annuloplastie ajustable (1) selon l'une des revendications précédentes, **caractérisé en ce que** la base (4) comprend des ancres (8) pour engager un anneau et/ou pour relier la cage (2) à la base annulaire (4).

11. Dispositif d'annuloplastie ajustable (1) selon la revendication 9 ou 10, **caractérisé en ce que** les ancres (9, 20) sont réalisées à partir d'un alliage à mémoire de forme ou comprennent un tel alliage.

12. Dispositif d'annuloplastie ajustable (1) selon la revendication 11, **caractérisé en ce que** les ancres (8) comprennent des épingles (40) avec une partie de corps (42) s'étendant longitudinalement, de préférence courbée, et une pointe (41) de préférence acérée.

13. Dispositif d'annuloplastie ajustable (1) selon l'une des revendications 3 à 6 et l'une des revendications 9 à 12, **caractérisé en ce que** l'au moins un élément rigide (6) comprend une ouverture, dans laquelle les ancres (8) s'étendent à travers ou sont adaptées pour s'étendre à travers ladite ouverture.

14. Dispositif d'annuloplastie ajustable (1) selon l'une des revendications précédentes, **caractérisé en ce qu'**une extrémité distale de la cage (2) et/ou de la base (4) est espacée d'une section proximale de la cage (2) et/ou de la base (4).

15. Système de délivrance (3) comprenant un dispositif d'annulo-plastie (1) selon l'une des revendications précédentes, **caractérisé en ce que** le dispositif de délivrance (1) comprend en outre une ligne de transmission (18), de préférence un cathéter, pour ajuster la cage (2) et/ou la base annulaire (4), la ligne de transmission (18) comprenant de préférence une ouverture pour un cathéter à ballon (38).

16. Dispositif d'annuloplastie percutanée ajustable (1) selon l'une des revendications 1 à 14, comprenant :
une base (4) s'étendant longitudinalement, de forme essentiellement annulaire, avec un état déployé et un état non déployé, dans lequel la base (4) est flexible de sorte que la forme annulaire de la base (4) est ajustable pendant et/ou après l'implantation, **caractérisé en ce que** la base (4) est fabriquée à partir d'un alliage à mémoire de forme ou comprend un alliage à mémoire de forme de sorte qu'à l'état déployé lors de la libération d'un dispositif de délivrance, la base a une forme annulaire, de préférence une forme en D..

17. Dispositif d'annuloplastie ajustable (1) selon la revendication 16, **caractérisé en ce que**, la base comprend au moins un élément flexible (5) et au moins deux éléments rigides (6), qui sont reliés par l'élément flexible (5) dans lequel l'élément flexible (5) permet une expansion et/ou une compression le long d'une direction longitudinale de la base (4) .

18. Dispositif d'annuloplastie ajustable (1) selon la revendication 17, **caractérisé en ce que** la base (4) comprend des éléments (5, 6) alternativement rigides et flexibles.

19. Dispositif d'annuloplastie ajustable (1) selon l'une des revendications 17 à 18, **caractérisé en ce que** le au moins un élément rigide (6) est mobile disposé à l'intérieur de la base (4) de manière à pouvoir être poussé vers une zone annulaire interne (24).

20. Dispositif d'annuloplastie ajustable (1) selon l'une des revendications 17 à 19, **caractérisé en ce que** le au moins un élément rigide (6) comprend une interface, de préférence une ouverture, pour une connexion à une cage (2).

21. Dispositif d'annuloplastie ajustable (1) selon l'une des revendications 17 à 20, **caractérisé en ce que** l'élément rigide comprend un premier patin (31), qui s'étend le long d'un plan de la forme annulaire et un second patin (32) qui est incliné par rapport au premier patin (31), le second patin (32) définissant de préférence une zone annulaire intérieure (24) de la forme annulaire.

22. Dispositif d'annuloplastie ajustable (1) selon la revendication 17, **caractérisé en ce que** l'élément flexible comprend ou consiste en un fil, dans lequel le fil a une forme ondulée, et dans lequel le fil (52) comprend une première et une deuxième extrémité et chaque extrémité est reliée à une première surface d'un élément rigide (6).

23. Dispositif d'annuloplastie ajustable (1) selon l'une des revendications précédentes, **caractérisé en ce que** le dispositif d'annuloplastie (1) comprend au moins un, de préférence deux, ancrage commissural (9, 20) pour fixer la base (4) à un anneau.

24. Dispositif d'annuloplastie ajustable (1) selon la revendication 23, **caractérisé en ce que** les ancres commissurales (9, 20) sont fabriquées à partir d'un alliage à mémoire de forme ou comprennent un tel alliage de sorte qu'une fois qu'un manchon (21) recouvrant l'ancre commissurale (9, 20) est retiré, les ancres (9, 20) sont déployées et adaptées pour pénétrer dans le tissu natif de l'anneau afin de réaliser deux fixations commissurales.
